# EUROPEAN PATENT SPECIFICATION

(11) **EP 3 560 544 B1**
(45) Date of publication and mention of the grant of the patent: **18.05.2022**
(21) Application number: 18169570.1
(22) Date of filing: 26.04.2018
(51) Int. Cl.: A61M 35/00, A61M 1/00, A61B 17/32, A61K 9/70

(54) **MEDICAL DEVICE FOR NITROGEN MONOXIDE (NO)-ASSISTED WOUND DEBRIDEMENT**
MEDIZINISCHE VORRICHTUNG ZUM WUNDENDEBRIDEMENT MITHILFE VON STICKSTOFFMONOXID (NO)
DISPOSITIF MÉDICAL POUR LE DÉBRIDEMENT DE PLAIES ASSISTÉ AU MONOXYDE D'AZOTE (NO)

(43) Date of publication of application: 30.10.2019
(73) Proprietor: BSN medical GmbH, 22761 Hamburg (DE)
(72) Inventor: Kaufmann, Steffen, 21357 Barum (DE); Dybe, Verena, 22523 Hamburg (DE); Zimmermann, Sebastian, 24118 Kiel (DE); Schulze, Christian, 21255 Tostedt (DE); Schütz, Patrick, 12159 Berlin (DE)
(74) Representative: FARAGO Patentanwaltsgesellschaft mbH

(56) References cited:
- US-A1- 2004 122 447
- US-A1- 2007 065 473
- US-A1- 2008 206 364
- US-A1- 2009 108 777
- US-A1- 2010 150 991
- US-A1- 2010 152 683
- US-A1- 2010 160 871
- US-A1- 2011 033 437
- US-A1- 2013 196 951
- US-A1- 2013 261 534
- US-A1- 2013 330 244
- US-A1- 2014 336 557
- US-A1- 2016 143 660
- US-A1- 2016 296 655
- US-A1- 2018 000 503

## Description

### Field of the invention:

The invention relates to a mechanical wound debridement device delivering nitric oxide with a predetermined temporal pattern. The invention further relates to a kit comprising the wound debridement device and a NO-release activator and an presealed arrangement comprising the device or the kit of the invention.

### Background of the invention

Chronic wounds present a significant individual and societal burden in their cost of care and they reduce patient quality of life. Key components of wound care include measures as debridement, irrigation and wound cleaning. Appropriate care removes necrotic tissue and reduces wound bioburden to enhance wound healing. Physical cleaning with debridement and irrigation is of documented efficacy.

Debridement is the surgical excision of dead, devitalized, and contaminated tissue and/or the removal of foreign matter from a wound. Debridement is advantageous because it cleans the wound and lowers the bacterial count of wound tissues, thereby facilitating the healing process.

Some wound cleaning and debridement devices with relatively large dimensions harbor the risk of removing unintended tissue from the subject or damaging the unintended tissue. Furthermore, they require a massive and costly base unit which is difficult to transport. Hence, there is a need for tissue removal devices which have small dimensions and improved functionality over existing products and procedures which allow them to more efficiently remove wound plaque as well as tissue from the patient.

Wound debridement devices often include an irrigation system to provide pressurized streams of fluid to dislodge unwanted tissue and foreign matter from a wound and a suction system to collect the dislodged matter and irrigation fluid. These hand-held wound debridement systems generally provide an efficient and effective means of debriding a wound. However, the use of such systems can, disadvantageously, result in the splashing of irrigation fluid and body fluids from the patient onto, e. g. the healthcare professional operating the debridement device.

Mechanical wound debridement involves the use of dry gauze dressings, wet to dry gauze dressings, impregnated gauze/tulle dressings or a monofilament fibre pad to remove non-viable tissue from the wound bed. The mechanical wound debridement is thus performed by abrasive elements which can be supported by the use of an irrigation fluid. US 2004/122447 A1 discloses a device for mechanical debridement at a wound site on the body surface comprising a hollow debridement tip with bristles. US 2018/000503 A1 discloses a robotic device for mechanical debridement. US 2013/261534 A1 discloses a debridement brush in fluid combination with a reservoir to deliver a solution to a wound site. US 2016/143660 A1 discloses a device for mechanical debridement at a wound site comprising a carrier, an abrasive unit and a medicament or antimicrobial integrated in the device. US 2013/196951 A1, US 2013/330244 A1, US 2010/152683 A1, US 2014/336557 A1 and US 2016/296655 A1 disclose the use of nitric oxide and nitric oxide donors in the field of wound treatment and wound dressings.

Given the complex requirements of wound healing the devices of the prior art do not sufficiently prepare the wound for the healing process.

Hence, there is still a need for an improved mechanical wound debridement device for efficient, safe and healing-supportive debridement of acute and chronic wounds. The objective of the present invention thus is to provide a wound debridement device which overcomes at least one of the above-mentioned disadvantages.

This problem is solved by provision of a wound debridement device according to claim 1. Specific embodiments are subject matter of further dependent claims.

### Summary of the invention

In a first aspect the present invention provides a device for mechanical debridement at a wound site on the body surface Z as claimed in claim 1. The invention further relates to a kit as claimed in claim 12 and an arrangement as claimed in claim 13. Further embodiments of the invention are defined by the dependent claims.

The wound debridement device of the present invention has several advantages over wound care products known in the prior art.

In the first instance the abrasive unit cleans the wound and removes contaminated tissue, foreign matter and bacteria from the wound.

In the second instance this abrasive debridement leads to a better accessable wound surface on which the released nitric oxide can act on the wound surface in an optimal way.

The nitric oxide can be released concomitantly during the debridement process or in a delayed release manner that overlaps with the mechanical debridement or is following the delayed release.

Since the nitric oxide donor can be integrated within the device or externally filled into the reservoir of the device, a broad spectrum of NO donors and NO-releasing formulations can be used for the device of the invention allowing the right treatment for the right group of wounds and thus enables a personalized wound care.

By releasing an NO donor into the wound site, the device of the invention can be even used for a sustained post-treatment provision of nitric oxide and hence can enable a prolonged NO treatment.

For the abrasive unit, a broad spectrum of textile and fiber materials allows the selection of the optimal material with regard to costs, processability, abrasive properties, softness or liquid absorbance.

As a plastic/textile fabric the wound debridement device can be easily sterilized and stored for its safe use in wound cleansing and wound debridement.

Since the wound debridement device of the invention can be based on known abrasive materials such as textile materials or plastic fibers it can be easily produced in a costefficient manner.

Due to its specific properties the wound debridement device has the capability to achieve clinical benefits such as acceleration and improvement of the wound-healing and stimulation of the wound edges.

In sum, the wound debridement device enables a safe and easy-to-use wound treatment with high patient compliance.

### Detailed description of the invention

In one embodiment of the invention, the carrier is made of a material selected from the list consisting of plastic film, foil, sheet or web of fibrous material such as textile fabric, or a composite of different layers attached to form a unitary sheet or web.

Preferably, the carrier is made plastic or metal. Advantageously, the carrier is liquid impermeable so that any wound-derived debris or exudate or any device-related NO-releasing liquid can not contaminate the user or the environment.

In another preferred embodiment the carrier is a plate, a film, or a foil.

In a further embodiment the carrier is a cleaning attachment that is attachable to a hand-held device for wound debridement. The cleaning attachment is suitably driven by an electric motor and/or a sonotrode. In this embodiment the hand-held device comprises an ultrasound generating unit consisting of a frequency generator and a sonotrode.

The rigidity of the carrier can be adapted to the debridement requirements. For an irregular wound surface or a protruding part of the body, a flexible carrier is preferred for adapting to the respective contour. The flexible carrier can e.g. be formed of a web of fibrous material such as textile fabric, or a composite of different layers attached to form a unitary sheet or web.

For increased/harsh debridement effect, a rigid carrier is preferred so that the mechanical force can be directly applied to the wound site. In this embodiment a carrier plate is preferred.

The abrasive unit of the debridement device has an abrasive surface which is designed as abrasive fibers.

The source of the abrasive material of the abrasive unit can be natural, synthetic, semi-synthetic or biosynthetic source.

In one embodiment the abrasive fibers as such or building a brush head can be made from natural, semisynthetic or synthetic fibers. Hereby, the skilled person can select the fiber material from a broad range of known textile material and according the specific debridement requirements.

In a preferred embodiment of the invention the natural fibers are cellulose fibers such as cotton fibers.

When using semisynthetic fibers, the use of viscose fibers or acetate fibers is preferred.

In the field of the synthetic fibers it is preferred to use a polymer selected from polyester, polyacrylonitrile (PAN), polyethylene, polypropylene or polyamide.

In a preferred embodiment of the invention the fibers and preferably also the carrier layer are made from a hydrophilic fibers or filaments which may assist in the removal of excessive exudate from the wound. For the case that a further proximally located liquid absorbing layer is present, the hydrophilic loop fiber system and carrier layer assist in the transport of the exudate to the liquid absorbing layer.

The fibers can suitably be bi-component fibres. Several architectures can be hereby used such as a sheet/core architecture, a side-by side architecture, an island-in-the-sea architecture or a wedge-pie architecture.

The fibers or filaments can have a round cross-section. However, alternative crosssections, such as triangular, trilobal, polygonal, scalloped oval, lobular with lengthwise striations, dog-bone, concertina, star, Y-shaped, collapsed tubes, square with voids, cruciform, ribbon, bean or hexachannel can be used.

In a further embodiment the fibers or filaments could be hollow fibers or filaments.

The carrier layer of the device can comprise or consist of a nonwoven fabric, a woven fabric, a stich bonded fabric a net or a knitted fabric.

In certain embodiments the device comprises a reservoir holding a NO-releasing formulation. The reservoir is designed to absorb and temporarily retain the NO-releasing formulation or the NO-donor so that the released NO can be administered to the wound site. The architecture of the reservoir can vary depending on the application. For example, the reservoir may be a porous (with open and/or closed pores) material and could comprise or consist of a foam, a woven, a non-woven, a grid, a fine mesh or have a fibrous scaffolding. The reservoir may be formed of any suitable material that can serve its function. For example, the reservoir material may be a hydrophobic material such as polyurethane ester or PU-ether constructed as an open-cell, foam material. In another example, the material may be a hydrophilic material such as polyvinyl acetate or hydrophilic polyurethane, which can be constructed as a small, closed-cell foam. The degree of hydrophobicity or hydrophilicity can vary depending upon the particular application of the debridement device.

Alternatively, the reservoir is formed as a container, a bag, a recess or a cavity which is opened toward the treatment site.

The externally applicable NO-releasing formulation, which is preferably filled into the reservoir of the device, is selected from the list consisting of a liquid, capsules, a coating, a cream, an ointment, a liquid foam, a gel or a paste.

In one embodiment, the NO-releasing donor is an integral component of the device.

In an alternative embodiment the NO-releasing donor is a constituent of the NO-releasing formulation and which is externally added to fill the reservoir.

For activation of the NO donor, a broad range of activation mechanisms and activators can be used. For example, the NO-releasing donor can be activated by acidic milieu, aqueous liquid, moisture, enzymes, electrolysis, ultrasound waves, temperature or electromagnetic radiation in order to release the NO from said donor.

In a further embodiment the NO-releasing donor is activated by ultrasonic waves (so called sonolysis) in order to release the NO from the donor. This has the advantage that the ultrasonic waves fulfil two functions: On one side the ultrasonic waves support the removal of wound debris; on the other side, the ultrasonic waves induce the release of NO, so that two wound healing-improving activities are given. One example for a NO donor that sonolytically generates NO is S-nitroso glutathione. Hereby, the NO release can be enhanced by use of specific quinone derivatives (see Alegria et al. 2009: "Quinone-enhanced sonochemical production of nitric oxide from S-nitroso glutathione"; Ultrason. Sonochem. 16(1): 190 - 196).

In another embodiment the ultrasonic waves induce the release of NO from NOfilled compartments such as particles or bubbles, which are preferably micro or nano bubbles/particles.

In a preferred embodiment the NO-releasing donor is a pharmaceutically acceptable substance. This is of advantage when not only the NO is released to the wound site but also the NO donor leading to a subsequent NO release within the wound.

A broad spectrum of NO-releasing donors (synonymous to the term "NO donor" or "NOD") can be used for the device of the invention. In a preferred embodiment the NO-releasing donor is selected from the group consisting of photolabile NO-donor, pH-labile NO-donor, water-labile NO-donor and redox-sensitive NO-donor and preferably is a nitrite salt or a NO-eluting polymer.

### Photolabile NO-donor

Photolabile NO donors are known in the state of the art and familiar to the skilled person as they generate NO by irradiation with electromagnetic waves. In a preferred embodiment the photolabile NO donors are selected from the list consisting of organic nitrates, inorganic nitrates, nitrites, S-, N- or O-nitroso compounds, NO-metal compounds and NO-chelating substances.

Examples of photolabile NOD comprise diazeniumdiolates (e.g. US patents no. 7,105,502; 7,122,529; 6,673,338), trans[RuCl([15]aneN4)NO]²⁺, nitrosyl ligands, 6-nitrobenzo[a]pyrrole, S-nitroso-glutathione, S-nitroso-thiols, S-nitroso-N-acetyl-D-penicillamine (SNAP), Flu-DNB, Flu-DNB-DB, nitroaniline derivatives (see US 2013/0224083 A1), 2-methyl-2-nitrosopropane, imidazolyl derivatives, nitrate esters, hydroxyl nitrosamine, hydroxylamine, hydroxyurea or sodium nitroprusside.

Further examples are RuNOCl3 and K₂RuNOCl₅ which are water-soluble, ionic caged NO compounds. On irradiation in the range 300-350 nm they liberate NO rapidly at a high rate.

In on embodiment the device further comprises a light source for activating the photolabile NOD.

In the scope of the invention, a light source generates an electromagnetic radiation that contains the spectrum of the visible light, infrared light, and, in particular, the UV radiation. UV radiation comprises both UVA and UVB radiation in this context.

The type of irradiation required to induce the NO release is generally known to a person skilled in the art. It is feasible to use any electromagnetic radiation capable of degrading photo-labile NO derivatives while producing nitric oxide. For example, in the scope of the present invention, nitric oxide can be produced by means of photolytic cleavage using UVA radiation with wavelengths of, for example, 320 to 400 nm. However, it is also feasible to use electromagnetic radiation of any other wavelength, which, alone or with the aid of chemical, physical or biological procedures, induces a photolytic cleavage of NO-generating NODs.

In a device for the implementation of the process according to the invention, the electromagnetic radiation can be emitted by a light source that can be attached outside to the device or is integrated within the device. It is important that the light exposure of the compartment containing the photolabile NO donor is maximized with respect to the induced decomposition of the substance and/or release of nitrogen monoxide. The source of the electromagnetic radiation in this context can be an incandescent lamp or gas discharge lamp (low pressure- or high pressure-discharging) that is coated with corresponding fluorochromes, light-emitting diode (LED), organic light-emitting diode (OLED), LASER orany other source of electromagnetic radiation capable of generating NO from the corresponding chemical precursors and/or substrates.

In order to optimally cleave the photo-labile NO precursors that are present in the carrier medium, the light source can emit electromagnetic radiation of wavelengths from 100 to 2,000 nm or electromagnetic radiation of any other wavelength, which, alone or with the aid of chemical, physical or biological procedures, can induce a cleavage of photolabile NO donors and thus can induce the production of NO.

Accordingly, referring to photolytic cleavage, it is preferred to have the irradiation area of the device be made from a material that does not impair the properties of the energy of an electromagnetic radiation source that is required for optimum release of nitric oxide, due to its properties, generates the light properties required for light-induced release of nitric oxide optimizes them or, in the case of pH-dependent generation of NO, promotes and optimizes the pH-induced decomposition of the NO, being preferably nitrite.

The light used for irradiation of the photo-labile NO donor is in a wavelength range that depends on the respective NO donor. Accordingly, nitrites are irradiated, for photolysis with UV light, in a wavelength range between 320 and 400 nm, preferably between 340 and 380 nm, and particularly preferably at 365 nm. Referring to S-nitroso compounds, irradiation in the UVA range (i.e. with wavelengths between 315 and 380 nm) is preferred, but light with a wavelength of up to 1,000 nm can also lead to a significant decomposition rate in this context.

Notably, the optimum wavelength for photolysis depends strongly on the metal cation. Especially in the presence of transition metal ions, e.g. Cu²⁺, aqueous nitrite solutions can absorb light of significantly longer wavelengths than is the case with "pure" nitrite solutions and therefore the nitrite ion can also be cleaved by light of wavelengths of 400 - 450 nm and even other wavelengths ≥ 450 nm while releasing NO. Likewise, S- and N-nitrosated chemical compounds can also be cleaved by photolysis with electromagnetic radiation ≥ 400 nm while releasing NO due to the relatively weak binding energy between NO and the residual molecule.

### pH-labile NO donors

pH-labile NO donors are known in the state of the art and familiar to the skilled person as they generate NO by establishing an acidic pH value. In a preferred embodiment the pH-labile NO donors are selected from the list consisting of organic nitrates, inorganic nitrates, nitrites, S-, N- or O-nitroso compounds, NO-metal compounds and NO-chelating substances.

In a preferred embodiment of the invention, the pH-labile NO donors are selected from the group containing organic nitrates, inorganic nitrates, inorganic nitrites, organic nitrite esters such as alkyl nitrites, sulfur-, nitrogen- or oxygen-nitroso compounds, NO-metal compounds, and NO-chelating substances.

Examples of pH-labile NOD comprise inorganic nitrites, alkylnitrites such as isopentylnitrite, diazeniumdiolates (e.g. US patents no. 7,105,502; 7,122,529; 6,673,338), 1.substituted diazen-1-ium-1,2-diolates ("NONOates"), mesoionic oxatrazoles (e.g. GEA3162 or 1,2,3,4-oxoriazolium-5-amino-3-(3,4-dichlorophenyl)-chloride, trans[RuCI([15]aneN4)NO]2+, sodium trioxodinitrate (Angeli's salt), nitrosyl ligands, 6-nitrobenzo[a]pyrrole, S-nitroso-glutathione, S-nitroso-thiols, S-nitroso-N-acetyl-D-penicillamine (SNAP), nitroaniline derivatives (see US 2013/0224083 A1), 2-methyl-2-nitrosopropane, imidazolyl derivatives, nitrate esters, hydroxyl nitrosamine, hydroxylamine, hydroxyurea or sodium nitroprusside.

As an example, diazeniumdiolates, containing preferably the non-O-derivatized diazeniumdiolate functional group, are known to decompose by a first order mechanism in the presence of a proton source.

NaNO₂ is particularly preferred as NOD and can be used, in further preferred manner, together with a combination of ascorbic acid and Trolox as antioxidants in the carrier medium.

In this context, the concentration of the nitrite salts, relative to the total weight of the carrier medium or matrix containing them, can be up to 20% by weight, preferably between 0.25 and 10% by weight, particularly preferably between 3 and 7.5% by weight.

In an alternative embodiment, a nitrate salt that is subject to enzymatic conversion to the corresponding nitrite salt can be used just as well. In this context, nitrates of alkali or alkaline earth metals are preferably used as such. To name some examples: LiNO3, NaNO₃, KNO₃, RbNO₃, CsNO₃, FrNOs, Be(NO₂)₃, Mg(NO₂)₃, Ca(NO₂)₃, Sr(NO₂)₃, Ba(NO₂)₃ or Ra(NO₂)₃. In this context, the concentration of the nitrate salts, relative to the total weight of the carrier medium containing them, can be up to 20% by weight, preferably between 0.25 and 10% by weight, particularly preferably between 3 and 7.5% by weight.

In order to induce the NO release from the pH-labile NO donor, an acidic pH value must be established in the respective compartment whereby said pH value should be sufficiently low such that it induces cleavage of the pH-labile NO donor while producing NO. The actual pH value depends on the pH-lability of the NO donor and the desired period of time for generation of NO. The lower the pH value, the faster the NO will be generated by the device.

According to the invention, the pH value in the device can be between 0.0 and 6.9, preferably between 2.0 and 6.0, particularly preferably between 4.5 and 6.0, and in particular is 5.0. As mentioned above, the optimal pH value depends on the specific NO donor that is used and the intended reaction rate and can be adjusted appropriately by a person skilled in the art.

In an embodiment of the invention, the acidic medium required in the process for release of NO from the pH-labile NO donor is generated through the addition of an acid or of a buffer with an acidic pH value (i.e. with a pH < 7).

Numerous acids are available for use as acids for this purpose by a person skilled in the art. These comprise not only mineral acids such as HCI, H₂SO₄, H₃PO₄ or HNO₃, but also organic acids such as acetic acid, citric acid or lactic acid.

In a particular embodiment, the acid is an antioxidant as well, such as, for example, ascorbic acid or thiolactic acid, or an anti-oxidation synergist such as 1-hydroxyethan-1.1-diphosphonic acid or uric acid. By this means, no antioxidant needs to be present in step (a). The antioxidant is added in the form of an acid in step (b) and thus gets to be effective specifically at the point in time from which harmful or undesired radicals arise as a result of the NO donor being cleaved.

In another embodiment, the acid is present as a solid in the solid carrier medium and is dissolved, and thus can become deprotonatable, upon the addition of water. In this context, the acid can be present in the form of a powder, granulate, nanoparticles or acid groups situated on a polymer.

Numerous water-labile and moisture-labile NO donors are known from the prior art which a person skilled in the art can use in this context.

### Redox-sensitive NO donors

Several redox-sensitive NO donors are known from the prior art which a person skilled in the art can use in this context.

In a preferred embodiment of the invention, the pH-labile NO donors are selected from the group containing organic or inorganic nitrites, amino acids, NO-containing antibiotics or organic nitroso compounds.

As an example, a N-Nitroso-N-oxybenzaminine salt a can be used since they represent a class of compounds which yield nitric oxide and the corresponding nitroso benzene derivative on oxidation.

In one embodiment L-arginine could be used as a redox-sensitive NO donor which is a substrate of nitric oxide synthases, which are enzymes that catalyze the oxidation of L-arginine to NO and L-citrulline.

A further example are the antibiotics alanosine and dopastin which release NO in vivo by enzymatic oxidation.

When using redox-sensitive NO donors, the oxidation or reduction of said donor can be performed by an oxidation or reduction agent, with the use of enzymes or electrochemically through electrolysis.

### Water-labile or moisture-labile NO donor

Several water-labile or moisture-labile NO donors are known from the prior art which a person skilled in the art can use in this context. These donors release NO when coming into contact with water, e.g. the hydrolysis in aqueous media leads to a spontaneous NO release.

Examples for water-labile NO donors are diazeniumdiolate NO donors such as 1-[2-(carboxylato)pyrrolidin-1-yl]diazen-1-ium-1,2-diolate (PROLI/NO), or the ammonium salt of N-nitroso-N-phenylhydroxylamine (cupferron),

In a preferred embodiment the NO donor represents a stable form of NO storage which is activated by a physical stimulus (e.g. light) or a chemical stimulus (e.g. acid, water, enzyme, reducing or oxidizing agent) for releasing the NO. In case of the chemical stimuli the respective compound is denominated as NO-release activator.

In a preferred embodiment, the debridement device of the invention in its pretreatment status comprises the NO donor as being separated from the NO-release activator. At the start of the debridement treatment or during the treatment the NO-release activator is brought into contact with the NO donor to initiate the release of NO from the device to the wound site. This activation mechanism can be provided by different embodiments.

In a first embodiment the NO donor and the NO-release activator are contained in separate compartments with a barrier separating these compartments. The compartments are configured such that breaking or removing the barrier allows the NO donor to mix with the NO-release activator.

As an example, the device comprises composite fibers with a core electrical heater covered by a layer of hydrogel containing thermoresponsive drug carriers. The fibers are loaded with the NO donor and/or NO-release activator and can be individually addressed to enable on-demand release of these compounds with a controlled temporal profile (see Mostafalu et al.,: "A textile dressing for temporal and dosage-controlled drug delivery." Adv. Funct. Mater. 2017: 1702399) .

In a second embodiment the device only comprises the NO donor, whereby the NO-release activator is externally added to the device before or during the debridement treatment.

In a third embodiment a mixture of NO donor and NO-release activator, being preferably a NO-releasing formulation, is added externally to the reservoir of the device. Hereby, the NO donor and NO-release activator can be also present in two different compartments separated by a barrier.

In a fourth embodiment the device is configured to release the NO donor into the wound site whereby the NO donor is activated by a wound constituent such as water or an acidic milieu or wound-derived enzymes in order to release the NO within the wound.

There are several strategies for incorporating the NO donor within the device of the invention in order to be integrated within the device and thereby giving also rise to the compartments as disclosed above.

In a first strategy the NO-releasing donor can be encapsulated in a three-dimensional matrix with the capability to fixate the NO-releasing donor or enclose the NO-releasing donor in the matrix material.

In the second strategy the NO-releasing donor is coated onto a surface of a device component and preferably onto the abrasive surface of the abrasive unit.

In the fourth strategy the NO-releasing donor is given as nanoparticles or microparticles.

In the fifth strategy the NO-releasing donor is a NO-releasing polymer that is a constituent of fibers and preferably nanofibers forming the abrasive surface of the device.

In a preferred embodiment the NO donor containing compartment, being preferably a fiber, is configured to release the NO donor upon debridement-associated mechanical stress acting upon this compartment. This can be e.g. established by fibers which become brittle during the mechanical debridement and thereby release the NO donor.

### Antioxidants

In a preferred embodiment the device further comprises at least one antioxidants in order to remove the multiply oxidized nitrogen oxides, oxygen radical anions or hydroxyl radicals that arise during the generation of NO.

Based on their chemical mode of action, antioxidants are sub-classified as radical scavengers or reducing agents.

Often, chain reaction-like radical transfers take place during oxidation reactions involving organic compounds. In this context, substances with sterically hindered phenol groups take effect and produce inert, stable radicals in the course of these transfers, which do not keep on reacting such that the reaction cascade is terminated (radical scavenger). These include natural substances such as the tocopherols and synthetic substances such as butylhydroxyanisol (BHA), butylhydroxytoluene (BHT), and gallates. They are to be used, in particular, with non-polar carrier media.

Moreover, reducing agents with a very low standard redox potential of less than + 0.4 V (at pH 7.0 and 25°C) can be used as well. Typical representatives include ascorbic acid (-0.04 V at pH 7 and 25°C), sulfurous acid salts (+0.12 V at pH 7 and 25 °C), and certain organic sulfur-containing compounds (e.g. glutathione, cysteine, thiolactic acid), that can be used predominantly in hydrophilic carrier media.

In a preferred embodiment, the at least one antioxidant is capable of reducing HNO₂, which is present as NO donor in acidic medium, to NO. For this purpose, the antioxidant, as a reducing agent, must have a standard redox potential of less than +1.0362 Volt, preferably of less than + 0.5 Volt, particularly preferably of less than + 0.2 Volt, and even more particularly preferably of less than 0 Volt.

Expediently, the at least one antioxidant is capable of reducing the harmful NO₂ radical to the NO₂⁻ anion. For effective elimination of the NO₂ radical, the bimolecular reaction constant k of the at least one antioxidant should preferably be more than 1.0 x 10⁶ M⁻¹s⁻¹ and preferably be more than 1.0 × 10⁷ M⁻¹s⁻¹. Antioxidants that are suitable according to the invention as well as the corresponding reaction constants are disclosed in Kirsch et al., 2002 (Biol. Chem 383; 389 - 399, see Table 1). To name some examples: Captopril thiolate, caffeic acid, sinapinic acid, ferulic acid, lycopene, zeaxanthin, lutein, astaxanthin, canthaxanthin, arachidonate, Gly-Tyr dipeptide, tyrosine, purines and pyrimidines such as the nucleobases adenine, guanine, cytosine, thymine, uracil, and the corresponding derivatives and analogues thereof including the nucleosides and nucleotides containing them.

In a further embodiment, the NO-releasing formulation used according to the invention, which preferably is a liquid, contains not only the antioxidant, but an anti-oxidation synergist as well. Synergists support the effect of antioxidants, for example by regenerating spent antioxidants (so-called "redox cycling"). By complexing traces of metals (sodium EDTA) or by producing an oxidation-inhibiting pH value, synergists can reinforce the antioxidative effect of a radical scavenger or reducing agent. Typical examples of anti-oxidation synergists include EDTA, 1-hydroxyethane-1.1-diphosphonic acid, citric acid, fumaric acid, uric acid, and 2-(hydroxymethyl)-1,4-benzyldiol.

It is particularly preferable in the production process according to the invention to use ascorbate or ascorbic acid as the antioxidant.

A person skilled in the art is aware of numerous antioxidants that are capable of degrading or neutralizing multiply-oxidized nitrogen oxides, oxygen radical anions, hydroxyl radicals, or aqua-complexed electrons. A person skilled in the art will select these according to the respective composition of the carrier medium.

Antioxidants such as, for example, tocopherols, tocotrienols, tocomonoenols, Irganox^{®}, Irgafos^{®}, butylhydroxyanisol (BHA) and butylhydroxytoluene (BHT) are well-suited for apolar carrier media, such as, for example, apolar solvents, creams or gels.

Water-soluble vitamin E derivatives such as Trolox or alpha-AMG, organic sulfur-containing compounds such as glutathione, cysteine or thiolactic acid or organic acids such as ascorbic acid, alpha-lipoic acid, hydroxycinnamic acids such as p-cumaric acid, ferulic acid, sinapinic acid or caffeic acid, or hydroxybenzoic acids such as gallic acid, protocatechuic acid, syringic acid or vanillic acid are well-suited for polar media such as, for example, aqueous liquids or hydrogels.

Other preferred antioxidants comprise polyphenolic compounds such as anthocyanins, flavonoids and phytooestrogens.

In a further embodiment of the invention, the debridement device has one or more of the following characteristics:
- the device is a non-implantable device,
- the device is a portable device;
- the device is a hand-held device;
- the device is a disposable device, preferably integrated in a mechanical, ultra sound-based or electrical debridement system;
- the device further comprises an ultrasound source;
- the devices further comprise a motor for mechanical movement of the cleaning attachment;
- the device further comprises a liquid absorbing layer capable of absorbing wound exudate and/or debris;
- the device is capable of site specific delivery and controlled release of NO under physiological conditions;
- the device further comprises a liquid-impermeable top layer;
- the device comprises one or more further active agents;
- the device is adapted for the use in conjunction with negative pressure therapy;
- the device comprises a sensor for sensing a wound condition, the NO concentration and/or wound exudate or blood;
- the device comprises an activation indicator for indicating a NO-releasing condition;
- the device is antimicrobially equipped;
- the device further comprises a handle, a grip, a hand strap, or a pocket;
- the device is configured to release the NO-releasing donor, preferably together with its activator compound, onto the wound site for subsequent release of a therapeutic dosage of NO in said wound site; or
- the device is arranged for initial release, burst release, immediate release, delayed release or extended release such as a sustained release or controlled release of NO at therapeutic dosage.

In one embodiment the NO donor and/or the NO-release activator is contained within the abrasive unit. Hereby it is preferred that the abrasive unit is arranged to release the nitric oxide, the NO donor, the NO-release activator and/or one or more further substance upon mechanical stress of the abrasive surface or upon addition of an activating compound.

In a preferred embodiment the device is arranged for a delayed NO-release at therapeutic dosage following for 0 min to 72 hours after the start of the mechanical debridement.

In another embodiment of the invention the upper liquid-absorbing layer further comprises at least one antimicrobial active compound, preferably selected from the group consisting of bacteriocin like inhibitory substance (BLIS), silver or copper-based compound, biguanide salt like polyhexamethylbiguanide (PHMB), chlorhexidine, phenol derivatives, such as thymol and eugenol, chlorophenol and chlordiphenyl ether.

The wound debridement device can be provided with additional layers such as, e.g., a covering layer as a liquid impermeable layer, preventing the contamination of the user and the environment and also the penetration of dirt and bacteria into the wound debridement device or even the wound.

This aim can be achieved by, for example, applying a fluid impermeable continuous protective film (hereinafter also referred to as backing film) as cover layer, whereby in a practical manner the backing film is water vapour-permeable. This layer which, as described above, should typically be impermeable to bacteria, adjoins the distal surface of liquid-absorbing layer. In an advantageous embodiment of the invention the film is only bound to the distal surface of the absorbing layer in a manner so that the film penetrates into the pores, cells or other intermediate spaces. The cover layer can be transparent to allow the level of filling or moisture in the wound debridement device or the status of the wound to be assessed without having to remove the dressing. The cover layer can be filled with coloring agents. In general, the film has a thickness of 10-500 µm and typically 15 to 45 µm, whereby film thicknesses of 30 ± micrometers are used in particular.

Films of this type are known from the prior art and comprise, for example, polyethylene-, polypropylene-, polyester-, polyurethane- or polyamide-based films.

Preferred is the use of polyurethane-based films, such as a polyurethane film supplied by Coveris Advanced Coatings (Wrexham, UK) under the product name INSPIRE^{®}, or elastomer polyesters or mixtures of polyurethane with polyesters and/polyvinyl chloride and polyether amide block copolymers. Alternatively, the backing layer can be a water-repellent and water vapour-permeable polyurethane foam with essentially closed cells, such supplied, for example, by Scapa (Greater Manchester, UK) under the product name Medifix.

In a further embodiment the wound debridement device comprises proteolytic enzymes to hydrolyze peptide bonds, in order to facilitate the removal of non-viable tissue from the wound.

By way of non-limiting example suitable proteolytic enzymes include fibrinolysin, DNAfrom bovine pancreas, krill multienzyme complex, collagenase Clostridiopeptidase A from Clostridium histolyticum, Bromelain enzyme complex, streptokinase, streptodornase or sutilain.

In a further embodiment the wound debridement device comprises silver or copper as antimicrobial agents. These metals can be included in the form of pure silver or copper filaments/fibers or by synthetic fibers which are coated with a copper or silver coating. The copper/silver might be included in one or more of the substructures of the device, namely in the carrier layer, the abrasive unit and especially on the abrasive surface.

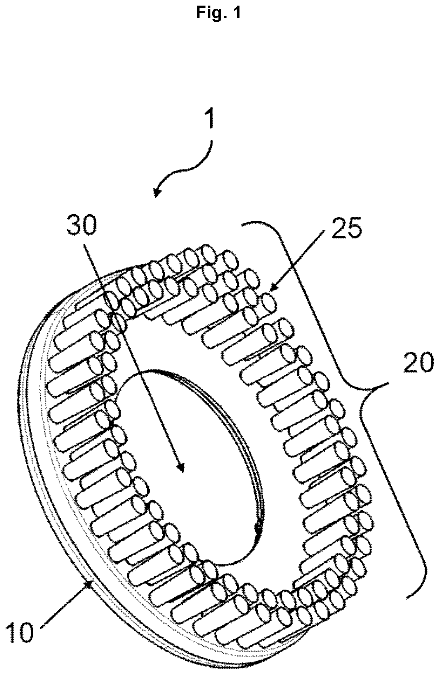

The dimension of the surface area of the wound debridement device may be selected according to the size of the wound but restricted to the possibility to use it as a manual device. Typically, a wound debridement device of the present invention may have a surface area of from 25 cm² to 400cm², and particularly of from 25 cm² to 200cm², such as 100cm². Also, the shape of the wound debridement device according to the present Z disclosure may vary with respect to the wound to be treated, and the present Z disclosure encompasses, for example, rectangular, square-, circle- or oval-shaped wound debridement device. The wound debridement device according to the present claimed invention has a circular shape as depicted in Fig. 1.

In one embodiment of the invention the wound debridement device further comprises a handle, a grip, a hand strap, or a pocket.

In a further aspect the invention relates to the use of the wound debridement device for removal of necrotic material, eschar, devitalized tissue, serocrusts, infected tissue, hyperkeratosis, slough, pus, hematomas, foreign bodies, debris, bone fragments or any other type of bioburden from a wound with the objective to promote wound healing.

In a further aspect the invention provides the device for the treatment of venous leg ulcers, diabetic foot ulcers (neuropathic and neuro-ischemic), arterial ulcers, mixed etiology ulcers, pressure ulcers, acute or traumatic wounds as well as incisions.

In another aspect the disclosure provides a process of wound debridement by use of the debridement device of the present invention encompasses the following steps:
1. Soaking or moistening the debridement device with the NO-releasing formulation or the formulation comprising the NO-release activator;
2. Wiping or rubbing the wound areal with the debridement device as prepared in step (a) with the abrasive surface facing the wound site for a time period that enables the release of NO and/NO donor in therapeutic doses into the wound site.

In a preferred embodiment of the device of the invention the release of NO and/NO donor in therapeutic doses by the device into the wound site is performed as a delayed release .

The delayed release is defined as a release that is initiated after the start of the debridement treatment. The delayed release could thus start at least 1, 2, 3, 4, 5, 6, 7, 8, 9, 10, 11, 12, 13, 14, 15, 20, 25 or even 30 minutes after the start of the treatment.

In a further embodiment the device is configured as a wound dressing, so that it can remain on the wound even after the debridement treatment. In this embodiment, the delayed release can even start at later time points such as at least 1, 2, 3, 4, 5, 6, 7, 8, 9, 10, 11, 12, 13, 14, 15, 20, 21 22, 23, 24, 25, 26, 27, 26, 29, 30, 31, 32, 33, 34, 35 or 36 hours after the start of the treatment.

In on embodiment of the disclosure the use of the wound debridement device of the invention includes also the liberation of wound edges and/or the liberation of peri-wound skin and thereby facilitates a subsequent wound closure.

In a further aspect, the invention provides a kit comprising at least one device according to the invention and a separate NO-release activator to be combined before or during debridement treatment.

In a further aspect, the invention provides an arrangement comprising at least one device according the invention packaged within a sealed package, whereby the sealed package is preferably an air-tight plastic or metal package and the at least one device is packaged in a sterile fashion and/or pre-moistured fashion.

### Brief description of the drawing:

These and other aspects of the invention will be apparent from and elucidated with reference to the embodiments described hereinafter.

The invention will now be described, by way of example, based on embodiments with reference to the accompanying drawing.

In the drawing:
Fig. 1 shows a principal sketch of the wound debridement device 1 according to a first embodiment in perspective view.
In Figure 1, like numbers refer to like objects throughout. Objects in the Figures are not necessarily drawn to scale.

### Detailed description of embodiments:

Various embodiments of the invention will now be described by means of the Figure.

Fig. 1 shows a principal sketch of the wound debridement device 1 according to a first embodiment in perspective view with a round plate as carrier 10 carrying an annular array of abrasive fibers 25 forming the abrasive unit 20. The abrasive unit 20 has a central opening in which a circular recess 30 is formed within said carrier plate acting as a reservoir for holding the NO-releasing formulation or the NO donor.

While the invention has been illustrated and described in detail in the drawings and the foregoing description, such illustration and description are to be considered illustrative or exemplary and not restrictive.

From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

### Definitions

As used herein, a "monofilament" is defined as a single untwisted synthetic filament.

The term "yarn" is defined as a long, continuous length of interlocked fibers. These fibers could be either natural, semi-synthetic fibers or filaments. The interlocking can be by twisting, plying or grouping together.

The "microfilaments" referred to herein are defined as continuous strands having a diameter in the region of about 17 to 20 µm or a linear density of between 0.3 and 1.0 dtex. The term dtex means decitex and is a unit expressing the linear density as the mass in grams of a 10,000 meter length of one filament. An alternative means of expressing the definition of a microfilament may be by reference to fiber thickness, which is generally expressed as a fiber diameter.

The term "nanofilaments" which is synonymous with the term "super-microfilaments" denotes to filaments with cross-sectional dimensions of less than 1 µm or a liner density of less than 0.3 dtex.

The term "cleansing" as used in the context of the present invention encompasses the debridement as well as the classical cleaning.

The term "debridement" as used herein refers to deeply removing adherent, dead or contaminated tissue from a wound and must be clearly separated from the act of classical cleaning, defined as the removal of dirt (loose metabolic waste or foreign material). Furthermore, debridement does not encompass revision of a wound, resection of functional tissue or amputation. Thus, debridement is defined as the act of removing necrotic material, eschar, devitalized tissue, serocrusts, infected tissue, hyperkeratosis, slough, pus, hematomas, foreign bodies, debris, bone fragments or any other type of bioburden from a wound with the objective to promote wound healing.

Debridement is sometimes referred to as a form of wound bed preparation; however, it has become clear that not only the wound bed but also the wound edges and the peri-wound skin are important for the successful healing of a wound. Hence, the present definition of the term "debridement" does not only refer to the removal of bioburden from the wound bed, but also the liberation of wound edges as well as of peri-wound skin.

The terms "proximal" and "distal" are used in the present invention to define the location of certain structures and especially layers in relation to the wound site. Proximal denotes to structures which are closer to the wound site, and distal to structures which are situated further away from the wound site.

The term "non-woven" is herein defined as sheet or web structures bonded together by entangling fiber or filaments mechanically, thermally or chemically. They are flat or tufted porous sheets that are made directly from separate fibers or filaments. Non-woven are not made by weaving or knitting and do not require converting the fibers to yarn.

### List of reference signs:

- 1: Wound debridement device
- 10: Carrier
- 20: annular abrasive unit
- 25: abrasive fibers
- 30: Recess as reservoir for holding NO-releasing formulation

## Claims

1. A device for mechanical debridement (1) at a wound site on the body surface comprising
A carrier (10),
An abrasive unit (20) forming an abrasive surface being sufficiently abrasive for mechanical removal of wound debris,
**characterized in that** the device for mechanical debridement further comprises:
a reservoir filled with an externally applicable NO-releasing formulation or NO-releasing donor configured to release a dosage of NO at said wound site on the body surface,
wherein the device has a round plate as carrier (10) carrying an annular array of abrasive fibers (25) forming the abrasive unit (20) which has a central opening in which a circular recess (30) is formed within said carrier plate acting as said reservoir filled with the NO-releasing formulation or the NO-releasing donor.

2. The device (1) according to claim 1, wherein the carrier (10) is made of a material selected from the list consisting of plastic film, foil, sheet or web of fibrous material such as textile fabric, or a composite of different layers attached to form a unitary sheet or web.

3. The device (1) according to claims 1 or 2, wherein the externally applicable NO-releasing donor is part of a NO-releasing formulation which is selected from the list consisting of a liquid, capsules, a coating, a cream, an ointment, a liquid foam, a gel or a paste.

4. The device (1) according to any of the above claims, wherein the NO-releasing donor is an integral component of said device or an externally added filling of said reservoir.

5. The device (1) according to any of the above claims, wherein the NO-releasing donor is activated by acidic milieu, aqueous liquid, moisture, enzymes, electrolysis, temperature, ultrasound waves or electromagnetic radiation in order to release the NO from said donor.

6. The device (1) according to any of the above claims, wherein the NO-releasing donor is selected from the group consisting of photolabile NO donor, acid-labile NO donor, water-labile NO donor, ultrasound-labile NO donor and redox-sensitive NO donor and preferably is a nitrite salt or a NO-eluting polymer.

7. The device (1) according to any of the above claims, wherein the NO-releasing donor is:
(a) encapsulated in a three-dimensional matrix with the capability to fixate the NO-releasing donor or enclose the NO-releasing donor in the matrix material; or
(b) given as nanoparticles or microparticles; or
(c) given as fibers and preferably as nanofibers of a NO-releasing polymer.

8. The device (1) according to any of the above claims, wherein the device has one or more of the following characteristics:
• the device is a non-implantable device,
• the device is a portable device;
• the device is a hand-held device;
• the device is a disposable device, preferably integrated in a mechanical, ultra sound-based or electrical debridement system;
• the device further comprises an ultrasound source;
• the device further comprises a motor for mechanical movement of the cleaning attachment;
• the device further comprises a liquid absorbing layer capable of absorbing wound exudate and/or debris;
• the device is capable of site specific delivery and controlled release of NO under physiological conditions;
• the device further comprises a liquid-impermeable top layer;
• the device comprises one or more further active agents;
• the device is adapted for the use in conjunction with negative pressure therapy;
• the device comprises a sensor for sensing a wound condition, the NO concentration and/or wound exudate or blood;
• the device comprises an activation indicator for indicating a NO-releasing condition;
• the device is antimicrobially equipped;
• the device further comprises a handle, a grip, a hand strap, or a pocket;
• the device is configured to release the NO-releasing donor, preferably together with its activator compound, onto the wound site for subsequent release of a therapeutic dosage of NO in said wound site; or
• the device is arranged for initial release, burst release, immediate release, delayed release or extended release such as a sustained release or controlled release of NO at therapeutic dosage.

9. The device (1) according to any of the above claims, wherein the device (1) is arranged for a delayed NO-release at a therapeutic dosage following for 1 min to 72 hours after the start of the mechanical debridement.

10. The device (1) according to any of claims 1 to 9 for the use for removal of necrotic material, eschar, devitalized tissue, serocrusts, infected tissue, hyperkeratosis, slough, pus, hematomas, foreign bodies, debris, bone fragments or any other type of bioburden from a wound with the objective to promote wound healing.

11. Device (1) according to claim 10 for the treatment of venous leg ulcers, diabetic foot ulcers (neuropathic and neuro-ischemic), arterial ulcers, mixed etiology ulcers, pressure ulcers, acute or traumatic wounds as well as incisions.

12. Kit comprising at least one device (1) according to any one of claims 1 to 11 and a separate NO-release activator to be combined upon debridement treatment.

13. Arrangement comprising at least one device (1) according to any one of claims 1 to 11 or at least one kit according to claim 12 packaged within a sealed package, whereby the sealed package is preferably an air-tight plastic or metal package and the at least one device or kit is packaged in a sterile fashion and/or pre-moistured fashion.

## Patentansprüche

1. Eine Vorrichtung zum mechanischen Debridement (1) an einer Wunde an der Körperoberfläche, die Folgendes umfasst:
einen Träger (10)
eine abrasive Einheit (20), die eine abrasive Oberfläche bildet, welche eine ausreichend abrasive Wirkung zum mechanischen Entfernen von Wunddebris hat,
**dadurch gekennzeichnet, dass** die Vorrichtung zum mechanischen Debridement weiter Folgendes umfasst:
ein Reservoir gefüllt mit einer äußerlich aufzutragenden NO-freisetzenden Formulierung oder einem NO-freisetzenden Donor, ausgebildet, um eine Dosierung von NO an der Wunde an der Körperoberfläche freizusetzen,
wobei die Vorrichtung eine runde Platte als Träger (10) hat, die eine ringförmige Anordnung von abrasiven Fasern (25) trägt, welche die abrasive Einheit (20) bildet, welche eine zentrale Öffnung hat, in der eine kreisförmige Vertiefung (30) innerhalb der Trägerplatte geformt ist, die als Reservoir wirkt, welches mit der NO-freisetzenden Formulierung oder dem NO-freisetzenden Donor gefüllt ist.

2. Die Vorrichtung (1) gemäß Anspruch 1, wobei der Träger (10) aus einem Material besteht, dass ausgewählt ist aus der Liste bestehend aus Kunststofffilm, Folie, Schicht oder Netz von faserigem Material, wie zum Beispiel Textilien oder einem Verbundstoff aus verschiedenen Schichten, die verbunden sind, um eine einheitliche Schicht oder ein einheitliches Netz zu bilden.

3. Die Vorrichtung (1) gemäß Anspruch 1 oder 2, wobei der äußerlich auftragbare NO-freisetzende Donor Teil einer NO-freisetzenden Formulierung ist, die ausgewählt ist aus der Liste bestehend aus einer Flüssigkeit, Kapseln, einer Beschichtung, einer Creme, einer Salbe, eines flüssigen Schaums, eines Gels oder einer Paste.

4. Die Vorrichtung (1) gemäß einem beliebigen der obigen Ansprüche, wobei der NO-freisetzende Donor ein integraler Bestandteil der Vorrichtung oder eine extern hinzugefügte Füllung des Reservoirs ist.

5. Die Vorrichtung (1) gemäß einem beliebigen der obigen Ansprüche, wobei der NO-freisetzende Donor aktiviert wird durch ein saures Milieu, eine wässrige Flüssigkeit, Feuchtigkeit, Enzyme, Elektrolyse, Temperatur, Ultraschallwellen oder elektromagnetische Strahlung, um das NO aus dem Donor freizusetzen.

6. Die Vorrichtung (1) gemäß einem beliebigen der obigen Ansprüche, wobei der NO-freisetzende Donor ausgewählt ist aus der Gruppe bestehend aus einem photolabilen NO-Donor, einem Säure-labilen NO-Donor, einem Wasser-labilen NO-Donor, einem Ultraschall-labilen NO-Donor und einem Redox-sensitiven NO-Donor und vorzugsweise ein Nitritsalz oder ein NO-eluierendes Polymer ist.

7. Die Vorrichtung (1) gemäß einem beliebigen der obigen Ansprüche, wobei der NO-freisetzende Donor Folgendes ist:
(a) eingekapselt in eine dreidimensionale Matrix, mit der Fähigkeit den NO-freisetzenden Donor zu fixieren oder in das Matrixmaterial einzuschließen; oder
(b) die Form von Nanopartikeln oder Mikropartikeln hat; oder
(c) die Form von Fasern und vorzugsweise Nanofasern eines NO-freisetzenden Polymers hat.

8. Die Vorrichtung (1) gemäß einem der obigen Ansprüche, wobei die Vorrichtung eine oder mehrere der folgenden Eigenschaften hat:
• die Vorrichtung ist eine nicht-implantierbare Vorrichtung;
• die Vorrichtung ist eine tragbare Vorrichtung;
• die Vorrichtung ist eine Vorrichtung, die in der Hand gehalten werden kann;
• die Vorrichtung ist ein Wegwerfvorrichtung, vorzugsweise integriert in ein mechanisches ultraschallbasiertes oder elektrisches Debridementsystem;
• die Vorrichtung umfasst weiter eine Ultraschallquelle;
• die Vorrichtung umfasst weiter einen Motor zur mechanischen Bewegung des Reinigungsaufsatzes;
• die Vorrichtung umfasst weiter eine flüssigkeitsabsorbierende Schicht, die in der Lage ist, Wundexsudat und/oder Debris zu absorbieren;
• die Vorrichtung ist fähig zu positionsgenauer Abgabe und kontrollierter Freisetzung von NO unter physiologischen Bedingungen;
• die Vorrichtung umfasst weiter eine flüssigkeitsundurchlässige Schicht;
• die Vorrichtung umfasst eine oder mehrere weitere aktive Substanzen;
• die Vorrichtung ist geeignet zur Verwendung in Verbindung mit Unterdrucktherapie;
• die Vorrichtung umfasst einen Sensor, zur Erfassung eines Wundzustands, der NO-Konzentration und/oder von Wundexsudat oder Blut;
• die Vorrichtung umfasst einen Aktivierungsindikator, zur Anzeige eines NO-freisetzenden Zustands;
• die Vorrichtung ist antimikrobiell ausgestattet;
• die Vorrichtung umfasst weiter einen Griff, einen Handgurt oder eine Tasche;
• die Vorrichtung ist konfiguriert, um den NO-freisetzenden Donor vorzugsweise gemeinsam mit seiner Aktivatorverbindung auf die Wunde freizusetzen, zur anschließenden Freisetzung einer therapeutischen Dosierung von NO in der Wunde; oder
• die Vorrichtung ist ausgebildet zur anfänglichen Freisetzung, schnellen Freisetzung, sofortigen Freisetzung, verzögerten Freisetzung oder verlängerten Freisetzung, wie zum Beispiel einer nachhaltigen Freisetzung oder kontrollierten Freisetzung von NO bei therapeutischer Dosierung.

9. Die Vorrichtung (1) gemäß einem beliebigen der obigen Ansprüche, wobei die Vorrichtung (1) ausgebildet ist für eine verzögerte NO-Freisetzung bei therapeutischer Dosierung, für 1 Minute bis 72 Stunden nach Beginn des mechanischen Debridements.

10. Die Vorrichtung (1) gemäß einem beliebigen der Ansprüche 1 bis 9 zur Verwendung zum Entfernen von nekrotischem Material, Schorf, devitalisiertem Gewebe, Serokrusten, infiziertem Gewebe, Hyperkeratose, abgestreifter Haut, Eiter, Hämatomen, Fremdkörpern, Debris, Knochenfragmenten oder jeder anderen Art von Keimbelastung einer Wunde mit dem Ziel die Wundheilung zu fördern.

11. Vorrichtung (1) gemäß Anspruch 10, zur Behandlung venöser Beingeschwüre, diabetischer Fußgeschwüre (neuropathisch und neuroischämisch), arterieller Geschwüre, Geschwüre mit gemischter Ätiologie, Druckgeschwüre, akuter oder traumatischer Wunden, sowie Schnitte.

12. Kit das eine Vorrichtung (1), gemäß einem beliebigen der Ansprüche 1 bis 11 und einen separaten NO-Freisetzungsaktivator umfasst, zu Kombination bei Debridementbehandlung.

13. Aufbau der Folgendes umfasst: mindestens eine Vorrichtung (1) gemäß einem beliebigen der Ansprüche 1 bis 11 oder mindestens ein Kit gemäß Anspruch 12, verpackt in einer verschlossenen Verpackung, wobei die verschlossene Verpackung vorzugsweise eine luftdichte Kunststoff- oder Metallverpackung ist und die mindestens eine Vorrichtung oder das mindestens eine Kit steril und/oder vorbefeuchtet verpackt ist.

## Revendications

1. Dispositif pour le débridement mécanique (1) sur un site de plaie sur la surface du corps comprenant
un support (10),
une unité abrasive (20) formant une surface abrasive étant suffisamment abrasive pour le retrait mécanique des débris de plaie,
**caractérisé en ce que** le dispositif pour le débridement mécanique comprend en outre :
un réservoir rempli d'une formule de libération de NO ou d'un donneur de libération de NO applicable de manière externe pour libérer une dose de NO sur ledit site de plaie sur la surface du corps,
dans lequel le dispositif présente une plaque ronde en tant que support (10) portant une rangée annulaire de fibres abrasives (25) formant l'unité abrasive (20) qui présente une ouverture centrale dans laquelle un creux circulaire (30) est formé à l'intérieur de ladite plaque porteuse agissant en tant que ledit réservoir rempli de la formule de libération de NO ou du donneur de libération de NO.

2. Dispositif (1) selon la revendication 1, dans lequel le support (10) est fait d'un matériau sélectionné à partir de la liste consistant en les films plastique, feuilles, films ou voiles de matériau fibreux comme du tissu textile ou un composite de différentes couches fixées pour former un film ou un voile unitaire.

3. Dispositif (1) selon la revendication 1 ou 2, dans lequel le donneur de libération de NO applicable de manière externe fait partie d'une formule de libération de NO qui est sélectionnée parmi la liste consistant en du liquide, des capsules, un revêtement, une crème, un onguent, une mousse liquide, un gel ou une pâte.

4. Dispositif (1) selon l'une quelconque des revendications ci-dessus, dans lequel le donneur de libération de NO est une composante intégrante dudit dispositif ou un remplissage ajouté de manière externe dudit réservoir.

5. Dispositif (1) selon l'une quelconque des revendications ci-dessus, dans lequel le donneur de libération de NO est activé par un milieu acide, un liquide aqueux, de l'humidité, des enzymes, une électrolyse, une température, des ondes ultrasoniques ou un rayonnement électromagnétique afin de libérer le NO dudit donneur.

6. Dispositif (1) selon l'une quelconque des revendications ci-dessus, dans lequel le donneur de libération de NO est sélectionné parmi le groupe consistant en un donneur de NO photo-labile, un donneur de NO acidelabile, un donneur de NO eau-labile, un donneur de NO ultrasons-labile et un donneur de NO sensible au redox et est de préférence un sel de nitrite ou un polymère d'élution de NO.

7. Dispositif (1) selon l'une quelconque des revendications ci-dessus, dans lequel le donneur de libération de NO est :
(a) encapsulé dans une matrice tri-dimensionnelle ayant la capacité de fixer le donneur de libération de NO ou d'enchâsser le donneur de libération de NO dans le matériau de matrice ; ou
(b) donné en tant que nanoparticules ou microparticules ; ou
(c) donné en tant que fibres et de préférence en tant que nanofibres d'un polymère de libération de NO.

8. Dispositif (1) selon l'une quelconque des revendications ci-dessus, dans lequel le dispositif présente une ou plusieurs des caractéristiques suivantes :
• le dispositif est un dispositif non-implantable ;
• le dispositif est un dispositif portable ;
• le dispositif est un dispositif manuel ;
• le dispositif est un dispositif jetable, de préférence intégré dans un système de débridement mécanique, à base d'ultrasons ou électrique ;
• le dispositif comprend en outre une source d'ultrasons ;
• le dispositif comprend en outre un moteur pour le mouvement mécanique de la fixation de nettoyage ;
• le dispositif comprend en outre une couche d'absorption de liquides capable d'absorber des exsudats et/ou débris de plaie ;
• le dispositif est capable de fourniture spécifique sur site et de libération contrôlée de NO dans des conditions physiologiques ;
• le dispositif comprend en outre une couche de dessus imperméable aux liquides ;
• le dispositif comprend un ou plusieurs agents actifs supplémentaires ;
• le dispositif est adapté pour l'utilisation en conjonction avec un traitement par pression négative ;
• le dispositif comprend un capteur pour détecter un état de la plaie, la concentration de NO et/ou de l'exsudat de plaie ou du sang ;
• le dispositif comprend un indicateur d'activation pour indiquer un état de libération de NO ;
• le dispositif est antimicrobien ;
• le dispositif comprend en outre un manche, une poignée, une sangle à main ou une poche ;
• le dispositif est configuré pour libérer le donneur de libération de NO, de préférence conjointement avec son composé activateur, sur le site de la plaie pour la libération consécutive d'une dose thérapeutique de NO dans ledit site de la plaie ; ou bien
• le dispositif est agencé pour la libération initiale, la libération par salves, la libération immédiate, la libération retardée ou la libération étendue telle qu'une libération durable ou une libération contrôlée de NO à dosage thérapeutique.

9. Dispositif (1) selon l'une quelconque des revendications ci-dessus, dans lequel le dispositif (1) est agencé pour une libération de NO retardée à une dose thérapeutique suivante pendant 1 min. à 72 heures après le commencement du débridement mécanique.

10. Dispositif (1) selon l'une quelconque des revendications 1 à 9, destiné à être utilisé pour le retrait de matière nécrotique, d'escarres, de tissus dévitalisés, de séro-croûtes, de tissus infectés, d'hyperkératose, de tissus nécrosés mous, de pus, d'hématomes, de corps étrangers, de débris, de fragments osseux ou de n'importe quel autre type de charge microbienne provenant d'une plaie avec l'objectif de favoriser la guérison de la plaie.

11. Dispositif (1) selon la revendication 10 pour le traitement d'ulcères veineux de la jambe, d'ulcères de pied diabétique, d'ulcères (neuropathiques ou neuro-ischémiques), d'ulcères artériels, d'ulcères d'étiologie mixte, d'ulcères de pression, de plaies graves ou traumatiques ainsi que des incisions.

12. Kit comprenant au moins un dispositif (1) selon l'une quelconque des revendications 1 à 11 et un activateur de libération de NO séparé destiné à être combiné lors du traitement par débridement.

13. Agencement comprenant au moins un dispositif (1) selon l'une quelconque des revendications 1 à 11 ou au moins un kit selon la revendication 12 emballé à l'intérieur d'un emballage fermé de manière étanche, ce par quoi l'emballage fermé de manière étanche est de préférence un plastique étanche à l'air ou un emballage métallique et l'au moins un dispositif ou kit est emballé de manière stérile et/ou de manière pré-humidifiée.
